# EUROPÄISCHE PATENTSCHRIFT

(11) **EP 0 354 354 B1**
(45) Veröffentlichungstag und Bekanntmachung des Hinweises auf die Patenterteilung: **27.04.1994**
(21) Anmeldenummer: 89112443.0
(22) Anmeldetag: 07.07.1989
(51) Int. Cl.: C07K 3/20, A61L 2/00, A61K 35/16

(54) **Verfahren zur Anreicherung der Blutgerinnungsfaktoren II, VII, IX und X**
Process for concentrating blood coagulation factors II, VII, IX en X
Procédé de concentration des facteurs de coagulation du sang II, VII, IX en X

(30) Priorität: 06.08.1988 DE 3826792
(43) Veröffentlichungstag der Anmeldung: 14.02.1990
(73) Patentinhaber: Kraus, Michael, Dipl.-Biol., D-70771 Leinfelden-Echterdingen (DE); Möller, Wolfgang, Dipl.-Chem. Dr., 61440 Oberursel (DE); Eichentopf, Bertram, Pharmazeut Dr., 65812 Bad Soden (DE)
(72) Erfinder: Kraus, Michael, Dipl.-Bio., D-6000 Frankfurt/M. 1 (DE); Möller, Wolfgang, Dr., Dipl.-Chem., D-6370 Oberursel (DE); Eichentopf, Bertram, Dr., D-6232 Bad Soden (DE)
(74) Vertreter: Wolff, Hans Joachim, Dr.jur. Dipl.-Chem.

(56) Entgegenhaltungen:
- EP-A- 0 208 215
- EP-A- 0 303 329
- WO-A-83/03760
- US-A- 4 508 709

## Beschreibung

Die Erfindung betrifft ein Verfahren zur Anreicherung der Blutgerinnungsfaktoren II, VII, IX und X in Zubereitungen aus Plasma oder anderen Fraktionen durch Adsorption der Gerinnungsfaktoren an eine alpha-Hydroxyl-Aminogruppen tragende Matrix und Elution der Faktoren mit dem Ziel der Bereitstellung von Zubereitungen höherer spezifischer Aktivität.

Nach der Hämophilie A ist die Hämophilie B die zweithäufigste vererbbare Blutgerinnungsstörung. Den Patienten fehlt der für die Gerinnung notwendige Faktor IX. Durch Substitution mit Faktor IX-haltigen Präparationen lässt sich diese vererbbare Gerinnungsstörung behandeln. Weitere Einsatzgebiete für die Faktoren II, VII, IX und X sind die erworbenen Blutgerinnungsstörungen wie zum Beispiel die disseminierte intravasale Gerinnung nach Polytraumatisierung oder der Mangel an den Vitamin K-abhängigen Faktoren bei Lebererkrankungen.

Bis 1954 stand für die Therapie des Faktor IX-Mangels nur fresh-frozen Plasma zur Verfügung. In den folgenden Jahren wurde durch Adsorption an Bariumsulfat (Aggeler, PM et al, Trans. 6th Congress, Internat. Soc. Hematol., Grune & Stratton, NY, 490-497, 1956) oder an Tricalziumphosphat (Janiak und Soulier, Thromb. et Diath. Haemorrh. 8, 406-424, 1962) versucht, den Faktor IX anzureichern.

Die Adsorption von Faktor IX an mineralische Adsorbentien wie z.B. Calziumphosphat, Bariumsulfat, Aluminiumhydroxyd oder Hydroxylapatit wurde im Laufe der Jahre weiter optimiert und zum Beispiel in der Europäischen Patentschrift 56629 beschrieben. Die spezifische Aktivität für Faktor IX lag dort bei 2,5 E pro mg Protein. Im gleichen Präparat war der Faktor II mit 2,0 E/mg Protein, der Faktor VII mit 1,0 E/mg Protein und der Faktor X mit 2,5 E/mg Protein vorhanden. Die Faktoren II, VII, IX und X werden auch als PPSB-Komplex bezeichnet.

Seit 1965 (Tullis et al., New Engl. J. Med. 273, 667-674, 1965) wird ebenfalls die DEAE-Anionenaustauschchromatographie zur Isolierung des Faktors IX bzw. des PPSB-Komplexes eingesetzt. Ein optimiertes Verfahren zur Isolierung des PPSB-Komplexes durch Adsorption an DEAE-Sephadex wird in der Europäischen Patentschrift 41174 beschrieben. Die spezifische Aktivität für Faktor IX betrug 2,2 E pro mg Protein im Endprodukt, der Faktor VII war hier stark abgereichert.

Faktor IX-Zubereitungen werden heute entweder als PPSB-Komplex mit den Faktoren II, VII, IX und X in wechselndem Verhältnis oder als hochangereichertes Faktor IX-Konzentrat für die Therapie von Gerinnungsstörungen eingesetzt.

Wenn alle vier PPSB-Faktoren in der Faktor IX-Zubereitung in etwa gleichem Verhältnis enthalten sein sollen, führt dies bei den dem heutigen Stand der Technik entsprechenden Adsorptionsverfahren aus Plasma zu einem Produkt mit einer spezifischen Aktivität für die Faktoren von rund 1 E/mg Protein, es sei denn, es wurde eine aufwendige mehrstufige Reinigungsmethode mit entsprechend niedriger Ausbeute angewendet.

Das zur Zeit am höchsten angereicherte PPSB-Handelsprodukt Prothromplex S-TIM 4 (Fa Immuno) kann eine spezifische Aktivität für alle vier Faktoren von mehr als 1 E/mg Protein nur durch separate Aufreinigung des Faktors VII und nachträgliches Zumischen zu dem PPSB-Komplex erreichen. Hochangereicherte Faktor IX-Konzentrate mit einer spezifischen Aktivität für Faktor IX von mehr als 10 E pro mg Protein lassen sich nach dem heutigen Stand der Technik nur durch mehrstufige Fraktionierungen aus Plasma gewinnen. Beispiele hierfür sind in der Internationalen Patentanmeldung WO 83/03760 oder in DE-3101752 beschrieben.

Der vorliegenden Erfindung lag die Aufgabe zu Grunde, ein einfaches Verfahren zur Gewinnung einer Faktor IX-Zubereitung mit mehr als 1 E Faktor IX pro mg Protein, die gegebenenfalls die anderen PPSB-Faktoren II, VII und X in etwa gleichem Verhältnis enthält, bereitzustellen.

Die Aufgabe wurde erfindungsgemäss dadurch gelöst, dass man Plasma oder eine andere die Gerinnungsfaktoren enthaltende Fraktion mit einer eine alpha-Hydroxyl-Amino-Gruppe tragenden Matrix versetzt und den Faktor IX, sowie gegebenenfalls die Faktoren II, VII und X adsorbiert, mit geeigneten Puffern nachwäscht und anschliessend die Faktoren eluiert. Die eine alpha-Hydroxyl-Amino-Gruppe tragende Matrix kann nach dem aus I. Matsumoto et al, J.

Biochem. 87, 535-540 (1980) bekannten Verfahren hergestellt werden.

Überraschenderweise wurde dabei gefunden, dass je nach der Ionenstärke der Lösung der Faktor IX oder der gesamte PPSB-Komplex sehr schnell und in hoher Ausbeute gebunden wird und mit einer hohen Reinheit wieder eluiert werden kann.

In der Literatur ist der Einsatz von Gelen mit Alkylamino-Gruppen ohne eine alpha-Hydroxyl-Amino-Gruppe für die Reinigung des Gerinnungsfaktors VIII bekannt. (Thromb. Haemostas. (Stuttgart) 47(2) 124-127 (1982); EP 0144957; Britisch Journal of Haematology, 1979, 43, 669-674). Diese Gele binden auch die PPSB-Faktoren. Die Adsorption folgt dabei in etwa den gleichen Gesetzmässigkeiten wie für Faktor VIII.

Das Adsorptionsverhalten solcher bekannter Gele (Sepharose) mit verschieden langen Kohlenstoffketten zwischen Matrix und endständiger Aminogruppe wurde in einem Vergleichsversuch untersucht und dem Adsorptionsverhalten eines Gels mit einer alpha-Hydroxyl-Aminopropylgruppe (alpha-Hydroxyl-Aminopropyl-Sepharose) gegenübergestellt. Dazu wurden jeweils 50 ml Citrat-Plasma mit Wasser im Verhältnis 1 : 3 verdünnt und über 1 x 5 cm Säulen, die mit verschiedenen Gelen gefüllt waren, chromatographiert. Der Gehalt der nicht gebundener Gerinnungsfaktoren im Restplasma wurde nach der Adsorption bestimmt und ist in der Tabelle 1 in Prozent der Aktivität des Ausgangsplasmas wiedergegeben.

**Tabelle 1**

| Restgehalt der PPSB-Faktoren im Plasma nach Adsorption an Amino-Sepharose | | | | | |
|---|---|---|---|---|---|
| | F.II | F.VII | F.VIII | F.IX | F.X |
| Amino-Ethyl-Sepharose | 99 | 97 | 93 | 97 | 98 |
| Amino-Propyl-Sepharose | 94 | 99 | 92 | 95 | 96 |
| Amino-Pentyl-Sepharose | 11 | 22 | 7 | 10 | 16 |
| Amino-Hexyl-Sepharose | 1 | 3 | 4 | 2 | 1 |
| Amino-Oktyl-Sepharose | 4 | 2 | 4 | 4 | 3 |
| alpha-Hydroxyl-Amino-Propyl-Sepharose (erfindungsgemäss) | 6 | 2 | 86 | 3 | 3 |

Aus der Tabelle 1 geht hervor, dass das Optimum für eine solche Adsorption an reine Amino-Alkyl-Träger ohne die alpha-Hydroxylamino-Gruppe bei einer Kettenlänge des sog. Spacers von 6 bis 8 C-Atomen liegt. Bei 3 C-Atomen werden nur geringe Mengen von den PPSB-Faktoren gebunden. Die Reinheit der PPSB-Faktoren nach einer Chromatographie über Amino-Hexyl-Sepharose ist in etwa mit der spezifischen Aktivität nach einer Chromatographie über eine DEAE-Matrix zu vergleichen.

Bei der Herstellung von Amino-Gruppen tragenden Trägern aus einer Epoxy-Vorstufe mit Ammoniak erhält man neben der Amino-Gruppe eine alpha-ständige Hydroxyl-Gruppe. Auf Grund der oben angeführten Ergebnisse für reine Alkylamino-Träger war es überraschend, dass nun die alpha-Hydroxyl-Amino-Träger ein völlig anderes Bindungsverhalten für die PPSB-Faktoren zeigen.

Im Gegensatz zu den Alkylamino-Trägern ist der Spacer mit 6 bis 8 C-Atomen nicht mehr notwendig. Selbst alpha-Hydroxyl-Amino-Gruppen an einem Spacer mit 3 C-Atomen führen zu einer starken Adsorption der PPSB-Faktoren.

Die PPSB-Faktoren werden wesentlich spezifischer adsorbiert und dann mit höherer Reinheit eluiert, als dies mit Alkylamino-Trägern und mit DEAE-Anionenaustauschern bisher möglich war.

Die Adsorption an alpha-Hydroxylamino-Träger erfolgt wesentlich schneller als an die bisher am häufigsten eingesetzten DEAE-Anionenaustauscher. Während die Adsorption der Faktoren II, VII, IX und X an DEAE-Sepharose zum Beispiel rund 1 bis 4 Stunden dauert, ist sie bei Verwendung der erfindungsgemässen alpha-Hydroxylaminopropyl-Gele und vergleichbarer Versuchsanordnung schon nach 5 Minuten vollständig.

Ein weiterer Unterschied zur bekannten DEAE-Anionenaustauschchromatographie ist die sehr gute Bindung von Faktor VII an die alpha-Hydroxylamino-Matrix bei niedriger Ionenstärke und die gemeinsame Elution aller PPSB-Faktoren mit annähernd gleich hoher spezifischer Aktivität von mehr als 1 E/mg Protein. Bei der DEAE-Anionenaustauschchromatographie verliert man bei solch hohen spezifischen Aktivitäten bisher zwangsläufig einen wesentlichen Anteil des Faktor VII. In der Praxis führte dies dazu, dass in solchen PPSB-Hochkonzentraten entweder der Faktor VII nur in geringerer Konzentration vorhanden war oder ein auf getrenntem Wege hergestellter Faktor VII zugemischt werden musste.

Die erfindungsgemässe Adsorption der PPSB-Faktoren an alpha-Hydroxyl-Amino-Träger erfolgt dabei vorzugsweie bei einem pH von 7,0 bis 7,5 und einer Konduktivität kleiner als 7,5 mS/cm. Durch Elution mit 0,25 bis 1 M NaCl lassen sich die PPSB-Faktoren mit einer Ausbeute von bis zu 80 % der Ausgangsaktivität gewinnen.

Als Ausgangsmaterial können sowohl Plasma als auch Plasmafraktionen oder andere die Gerinnungsfaktoren enthaltende Lösungen eingesetzt werden.

Das erfindungsgemässe Verfahren kann auch zur Gewinnung bzw. Anreicherung einzelner PPSB-Faktoren II, VII und X benutzt werden, wenn die Ausgangslösungen nur einen einzelnen Faktor enthalten.

Neben einer solchen angereicherten PPSB oder die genannten einzelnen Faktoren enthaltenden Zubereitung lässt sich durch geeignete Chromatographiebedingungen aus Plasma oder Plasmafraktionen mit den erfindungsgemässen alpha-Hydroxylamino-Trägern auch ein Faktor IX-Hochkonzentrat herstellen. Bei der Adsorption von Plasma oder Plasmafraktionen bei leicht erhöhter Konduktivität, vorzugsweise 12 bis 15 mS/cm, und einem pH von vorzugsweise 7,5 bis 7,7 bindet besonders der Faktor IX noch spezifischer an die Matrix und lässt sich mit einer Reinheit von 10 bis 20 E/mg Protein eluieren. Solche spezifischen Aktivitäten waren bisher nur durch mehrstufige Verfahren zu erreichen, deren Ausbeuten allerdings deutlich unter den 30-40 % liegen, die sich mit dem erfindungsgemässen Verfahren erreichen lassen. Die Adsorption der Faktoren II, VII, IX und X an alpha-Hydroxyl-Amino-Träger stellt damit einen wesentlichen Fortschritt bei der technischen Gewinnung des Gerinnungsfaktors IX oder des PPSB-Komplexes dar. Die Vorteile liegen in einer hohen Ausbeute und einer hohen Reinheit bei einem einfachen und schnellen Verfahren.

Die Adsorption der Gerinnungsfaktoren ist dabei nicht auf eine Säulenchromatographie beschränkt, sondern kann ebenso im Batchverfahren durchgeführt werden. Aufgrund der ausserordentlich schnellen Bindung der Faktoren an die Matrix eignet sich das erfindungsgemässe Verfahren auch für die Affinitätstrennung über Membranen, wenn eine mit alpha-Hydroxyl-Aminogruppen modifizierte Membran zur Adsorption der Faktoren eingesetzt wird.

Aus entsprechend vorgereinigten oder angereicherten Lösungen lassen sich auch die Gerinnungsfaktoren II oder VII oder X zu den entsprechenden Hochkonzentraten aufreinigen. Vor oder nach der Adsorption der Gerinnungsfaktoren an die alpha-Hydroxyl-Amino-Matrix kann man eine Sterilisation mit β-Propiolakton und/oder UV-Bestrahlung, mit Lösungsmittel/Detergentien oder durch Pasteurisierung durchführen, um eventuell vorhandene humanpathogene Viren zu inaktivieren.

Die nachstehenden Beispiele erläutern die Erfindung:

### Beispiel 1

Eine 25 ml Säule mit einer alpha-Hydroxyl-Amino-Propyl tragenden Matrix, bestehend aus einem Copolymerisat von Glycidylmethacrylat, Pentaerythrol-dimethacrylat und Polyvinylalkohol (Fraktogel-TSK-Amino der Firma Merck, Darmstadt) wurde mit 100 ml Puffer B (10 mM Citrat; pH 7,0) equilibriert.

350 ml Citrat-Plasma wurden auf eine Konduktivität von 7 mS/cm verdünnt und mit einer Flussrate von 300 ml/h auf die Säule aufgetragen. Anschliessend wurde mit 100 ml Puffer B gewaschen. Die PPSB-Faktoren wurden mit 0,5 M NaCl in Puffer B eluiert. Die Ausbeute für die PPSB-Faktoren lag bei 60 bis 80 % der Ausgangsaktivität. Die spezifische Aktivität der PPSB-Faktoren betrug 2,1 E F II/mg Protein, 1,9 E F VII/mg Protein, 2,3 F IX/mg Protein und 2,0 F X E/mg Protein.

### Beispiel 2

Eine 20 ml Säule mit dem in Beispiel 1 verwendeten Adsorbens wurde mit 50 ml Puffer A (10mM Citrat, 10mM NaHPO₄, 100mM NaCL, pH 7,5) equilibriert. 1000ml Plasma mit einer Konduktivität von 13 mS/cm wurden mit einer Flussrate von 300 ml/h aufgetragen und dann mit 150 ml Puffer A gewaschen. Der Faktor IX wurde mit einem linearen Gradienten von 0 bis 0,5M NaCl in Puffer A, der zusätzlich 0,5 E/ml AT III und 5 E/ml Heparin enthielt, eluiert.
In den Fraktionen wurde der Faktor IX- und Proteingehalt bestimmt.

Die Fraktionen mit einer spezifischen Aktivität von über 5 E Faktor IX/mg Protein wurden gepoolt.
Auf diese Weise wurde ein Faktor IX-Konzentrat erhalten, das eine spezifische Aktivität von 15 E Faktor IX / mg Protein aufwies. Die Ausbeute betrug 40% der Ausgangsaktivität im Plasma.

### Beispiel 3

Jeweils 200 ml Plasma wurden analog zu Beispiel 1 auf verschiedenen Trägern mit der alpha-Hydroxyl-Amino-Gruppe chromatographiert. Dabei handelte es sich um folgende Träger:
- Nr. 1: Copolymerisat von Vinylacetat und Divinylethylenharnstoff (z.B. Biosynth)
- Nr. 2: Copolymerisat von Methacrylamid, N-Methylen-bis-methacrylamid und Allylglycidyl-ether (z.B. Eupergit)
- Nr. 3: Polymeres von Kohlehydraten (z.B. Sepharose)
Die Ergebnisse waren prinzipiell die gleichen wie im Beispiel 1 und sind in der Tabelle 2 wiedergegeben.

**Tabelle 2**

| Träger Nr. | Ausbeute in % | | | | spez. Akt. E/mg | | | |
|---|---|---|---|---|---|---|---|---|
| | FII | FVII | FIX | FX | FII | FVII | FIX | FX |
| 1 | 50 | 82 | 55 | 61 | 1,5 | 2,1 | 1,5 | 1,7 |
| 2 | 57 | 70 | 54 | 48 | 1,6 | 2,0 | 1,8 | 1,5 |
| 3 | 52 | 43 | 57 | 46 | 1,2 | 1,0 | 1,3 | 1,1 |

### Beispiel 4

1000 ml Plasma wurden mit 0,25 % β-Propiolakton bei pH 8,0 über Nacht behandelt und anschliessend mit UV-Licht bestrahlt. Das kaltsterilisierte Plasma wurde analog zu Beispiel 1 aufgearbeitet. Bei einer Ausbeute von 60 bis 80 % war die Reinheit der PPSB-Faktoren mit der in Beispiel 1 angegebenen spezifischen Aktivität vergleichbar.

### Beispiel 5

100 ml eines PPSB-Handelsproduktes mit einer spezifischen Aktivität von 0,6 E Faktor IX/mg Protein wurden analog zu Beispiel 2 über 30 ml des in Beispiel 1 verwendeten Adsorbens chromatographiert. Auf diese Weise liess sich ein Faktor IX-Hochkonzentrat mit 10,4 E/mg Protein mit einer Ausbeute von 95 % isolieren.

## Patentansprüche

1. Verfahren zur Anreicherung eines oder mehrerer der Blutgerinnungsfaktoren II, VII, IX und X, dadurch gekennzeichnet, dass man eine die Faktoren enthaltende Lösung mit einem eine alpha-Hydroxyl-Amino-Gruppe tragenden Adsorbens adsorbiert, gegebenenfalls das Adsorbens wäscht und die Blutgerinnungsfaktoren eluiert.

2. Verfahren nach Anspruch 1, dadurch gekennzeichnet, dass man als Ausgangslösung den Blutgerinnungsfaktor enthaltendes Plasma, Plasmafraktionen oder Fermentationslösungen oder Fraktionen davon verwendet.

3. Verfahren nach Anspruch 1 und 2, dadurch gekennzeichnet, dass man als Träger für die alpha-Hydroxyl-Amino-Gruppe organische Polymere einsetzt.

4. Verfahren nach Anspruch 1 bis 3, dadurch gekennzeichnet, dass man als Träger Polymere vom Typ eines Copolymerisates von Glycidylmethacrylat, Pentaerythrol-dimethacrylat und Polyvinylalkohol einsetzt.

5. Verfahren nach Anspruch 1 bis 3, dadurch gekennzeichnet, dass man als Träger Polymere vom Typ eines Copolymerisates von Methacrylamid, N-Methylen-bis-methacrylamid und Allyl-glycidyl-ether einsetzt.

6. Verfahren nach Anspruch 1 bis 3, dadurch gekennzeichnet, dass man als Träger Polymere vom Typ eines Copolymerisates von Vinylacetat und Divinylethylen-Harnstoff einsetzt.

7. Verfahren nach Anspruch 1 bis 3, dadurch gekennzeichnet, dass man als Träger Polymere von Kohlehydraten einsetzt.

8. Verfahren nach Anspruch 1 und 2, dadurch gekennzeichnet, dass man als Träger für die alpha-Hydroxyl-Amino-Gruppe anorganische Polymere einsetzt.

9. Verfahren nach Anspruch 8, dadurch gekennzeichnet, dass man als Träger für die alpha-Hydroxyl-Amino-Gruppe eine Silikat-Matrix einsetzt.

10. Verfahren nach Anspruch 1 bis 9, dadurch gekennzeichnet, dass man die Adsorption als Säulenchromatographie, im Batchverfahren oder an Membranen durchführt.

11. Verfahren nach Anspruch 1 bis 10, dadurch gekennzeichnet, dass vor oder nach der Adsorption mit alpha-Hydroxyl-Amino-Trägern in der Lösung vorhandene Viren durch geeignete Massnahmen inaktiviert werden.

12. Verfahren nach Anspruch 11, dadurch gekennzeichnet, dass vor oder nach der Adsorption mit alpha-Hydroxyl-Amino-Trägern in der Lösung vorhandene Viren durch Behandlung mit β-Propiolakton und/oder UV-Bestrahlung, durch Behandlung mit Lösungsmitteln und/oder Detergentien oder durch Pasteurisation in Lösung inaktiviert werden.

## Claims

1. A process for increasing the concentration of one or more of the blood clotting factors II, VII, IX and X, characterised in that a solution containing the factors is adsorbed with an adsorbent carrying an α-hydroxyl-amino group, the adsorbent is optionally washed and the blood clotting factors are eluted.

2. A process according to Claim 1, characterised in that the starting solution used is plasma, plasma fractions or fermentation solutions or fractions thereof containing the blood clotting factor.

3. A process according to Claims 1 and 2, characterised in that the carriers used for the α-hydroxyl-amino group are organic polymers.

4. A process according to Claims 1 to 3, characterised in that the carriers used are polymers of the type of a copolymer of glycidyl methacrylate, pentaerythritol dimethacrylate and polyvinyl alcohol.

5. A process according to Claims 1 to 3, characterised in that the carriers used are polymers of the type of a copolymer of methacrylamide, N-methylene-bis-methacrylamide and allyl-glycidyl ether.

6. A process according to Claims 1 to 3, characterised in that the carriers used are polymers of the type of a copolymer of vinyl acetate and divinyl ethylene urea.

7. A process according to Claims 1 to 3, characterised in that the carriers used are polymers of carbohydrates.

8. A process according to Claims 1 and 2, characterised in that inorganic polymers are used as carriers for the α-hydroxyl-amino group.

9. A process according to Claim 8, characterised in that a silicate matrix is used as carrier for the α-hydroxyl-amino group.

10. A process according to Claims 1 to 9, characterised in that the adsorption is carried out as column chromatography, by the batch process or on membranes.

11. A process according to Claims 1 to 10, characterised in that before or after the adsorption with α-hydroxyl-amino carriers, viruses present in the solution are inactivated by suitable measures.

12. A process according to Claim 11, characterised in that before or after the adsorption with α-hydroxyl-amino carriers, viruses present in the solution are inactivated by treatment with β-propiolactone and/or UV irradiation, by treatment with solvents and/or detergents or by pasteurisation in solution.

## Revendications

1. Procédé de concentration d'un ou plusieurs des facteurs de coagulation sanguine II, VII, IX et X, caractérisé en ce qu'on adsorbe une solution contenant les facteurs à l'aide d'un adsorbant porteur d'un groupement α-hydroxylamine, qu'on lave éventuellement l'adsorbant et qu'on élue les facteurs de coagulation sanguine.

2. Procédé selon la revendication 1, caractérisé en ce qu'on utilise comme solution de départ du plasma des fractions plasmatiques ou des solutions de fermentation ou des fractions de celles-ci contenant le facteur de coagulation sanguine.

3. Procédé selon les revendications 1 et 2, caractérisé en ce qu'on utilise des polymères organiques comme porteurs du groupement α-hydroxylamine.

4. Procédé selon les revendications 1 à 3, caractérisé en ce qu'on utilise comme porteurs des polymères du type copolymère de méthacrylate de glycidyle, diméthacrylate de pentaérythrol et poly(alcool vinylique).

5. Procédé selon les revendications 1 à 3, caractérisé en ce qu'on utilise comme porteurs des polymères du type copolymère de méthacrylamide, N-méthylène-bis-méthacrylamide et éther allylglycidylique.

6. Procédé selon les revendications 1 à 3, caractérisé en ce qu'on utilise comme porteurs des polymères du type copolymère d'acétate de vinyle et de divinyléthylène-urée.

7. Procédé selon les revendications 1 à 3, caractérisé en ce qu'on utilise comme porteurs des polymères de glucides.

8. Procédé selon les revendications 1 et 2, caractérisé en ce qu'on utilise des polymères inorganiques comme porteurs du groupement α-hydroxylamine.

9. Procédé selon la revendication 8, caractérisé en ce qu'on utilise une matrice de silicate comme porteur du groupement α-hydroxylamine.

10. Procédé selon les revendications 1 à 9, caractérisé en ce qu'on procède à l'adsorption sous forme de chromatographie sur colonne, en discontinu ou sur des membranes.

11. Procédé selon les revendications 1 à 10, caractérisé en ce qu'on inactive les virus présents dans la solution par des mesures appropriées avant ou après l'adsorption sur les porteurs du groupement α-hydroxylamine.

12. Procédé selon la revendication 11, caractérisé en ce qu'on inactive les virus présents dans la solution, avant ou après l'adsorption sur les porteurs du groupement α-hydroxylamine, en solution, par traitement par la β-propiolactone et/ou par irradiation UV, par traitement par des solvants et/ou des détergents ou par pasteurisation.
